# EUROPEAN PATENT APPLICATION

(11) **EP 2 034 313 A1**
(43) Date of publication of application: **11.03.2009**
(21) Application number: 08015602.9
(22) Date of filing: 04.09.2008
(51) Int. Cl.: G01N 33/543

(54) **Method for measuring interaction between physiologically active substance and test substance**

(30) Priority: 05.09.2007 JP 2007229661
(71) Applicant: Fujifilm Corporation, Tokyo 106-0031 (JP)
(72) Inventor: Kuruma, Koji, Tokyo 106-0031 (JP); Ezoe, Toshihide, Woodbridge CT 06525 (US)
(74) Representative: HOFFMANN EITLE

(57) **Abstract**

It is an object of the present invention to provide a method for measuring the interaction between a physiologically active substance immobilized on the surface of a substrate and a test substance, wherein the influence of negative signals intermittently generated due to desorption of the physiologically active substance as a ligand from the surface of the substrate upon the measured value that indicates the binding amount of a test substance as an analyte can be eliminated. The present invention provides a method for measuring the interaction between a physiologically active substance immobilized on the surface of a substrate and a test substance, wherein a calibration curve is produced by using baseline values obtained by repeatedly measuring a single type of solution, and the measurement value of said test substance is calibrated by said calibration curve, so as to obtain the interaction signals of said test substance.

## Description

### TECHNICAL FIELD

The present invention relates to a method for measuring the interaction between a physiologically active substance immobilized on the surface of a substrate and a test substance.

### BACKGROUND ART

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles. The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). In addition, the ultra-fine particle surface (nm level) of gold is functionalized, and physiologically active substances are immobilized thereon. Thus, a reaction to recognize specificity among physiologically active substances is carried out, thereby detecting a substance associated with a living organism from sedimentation of gold fine particles or sequences.

In all of the above-described techniques, the surface where a physiologically active substance is immobilized is important. Surface plasmon resonance (SPR), which is most commonly used in this technical field, will be described below as an example.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules. For example, Japanese Patent No.2815120 discloses a measurement chip where dextran is bound to gold substrate via self-assembled membrane (SAM) and carboxyl group is introduced into the dextran.

In general, in an apparatus of immobilizing a ligand on the surface thereof so as to detect an analyte binding to the ligand as a signal, there is a case where immobilization of the ligand on the surface is insufficient. In such a case, due to desorption of the ligand from the surface, negative signals are intermittently generated. Such negative signals affect detection of the bond of the analyte, and there are cases where only a signal amount that is lower than the value that should really be obtained is obtained. In particular, when a low molecular weight analyte binding to a ligand protein is detected in an apparatus for detecting a change in the refractive index of a near field, such as SPR, since signals depend on the molecular weight, desorption of a single molecule of ligand may be equivalent to 100 molecules of analyte-binding signals, and thus binding signals result in a negative value. This is problematic.

### DISCLOSURE OF THE INVENTION

It is an object of the present invention to solve the aforementioned problem of the prior art technique. Namely, it is an object of the present invention to provide a method for measuring the interaction between a physiologically active substance immobilized on the surface of a substrate and a test substance, wherein the influence of negative signals intermittently generated due to desorption of the physiologically active substance as a ligand from the surface of the substrate upon the measured value that indicates the binding amount of a test substance as an analyte can be eliminated.

As a result of intensive studies directed towards achieving the aforementioned object, the present inventors have found that, in a biosensor for measuring the interaction between a physiologically active substance immobilized on the surface of a substrate and a test substance, the aforementioned influence of negative signals intermittently generated due to desorption of the physiologically active substance from the surface of the substrate upon the measured value that indicates the binding amount of a test substance as an analyte can be eliminated by producing a calibration curve using baseline values obtained by repeatedly measuring a single type of solution and then calibrating the measurement value of the above-described test substance in the above-described calibration curve, thereby completing the present invention.

Thus, the present invention provides a method for measuring the interaction between a physiologically active substance immobilized on the surface of a substrate and a test substance, wherein a calibration curve is produced by using baseline values obtained by repeatedly measuring a single type of solution, and the measurement value of said test substance is calibrated by said calibration curve, so as to obtain the interaction signals of said test substance.

Preferably, the calibration curve is a curve obtained by approximating the baseline values by the least square method.

Preferably, the calibration curve is a first exponential curve (a linear approximation), a second exponential curve, a third exponential curve or an exponential approximation.

Preferably, the number of the baseline values used in production of the calibration curve is between 4 and 1,000.

Preferably, the interaction between the physiologically active substance and the test substance is detected as a change in refractive index.

Preferably, the interaction between the physiologically active substance and the test substance is detected by surface resonance plasmon analysis.

According to the method of the present invention, the precision of positive signals can be improved by continuously supplying an analyte to the surface of a single substrate on which a ligand is immobilized, measuring the bond of the analyte to the ligand, and producing a calibration curve while taking into consideration a fluctuation in the base signal of each analyte, and then calibrating the analyte signal value.

### BRIEF DESCRIPTION OF THE DRAWINGS

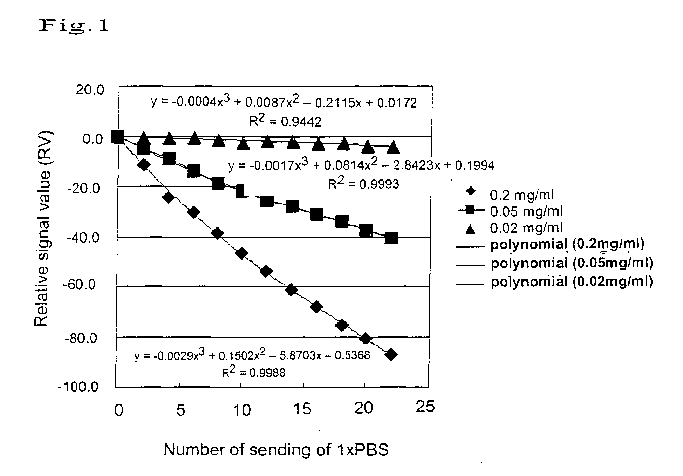

Figure 1 shows the results obtained by plotting 12 absolute values of signals in each buffer (1 x PBS) obtained in Comparative example 1.

### BEST MODE FOR CARRYING OUT THE INVENTION

The method of the present invention relates to a method for measuring the interaction between a physiologically active substance immobilized on the surface of a substrate and a test substance, which is characterized in that a calibration curve is produced by using baseline values obtained by repeatedly measuring a single type of solution, and the measurement value of said test substance is calibrated by said calibration curve, so as to obtain the interaction signals of said test substance.

When the measurement method of the present invention is carried out, a solution containing a test substance and a solution containing no test substances are alternatively supplied to the surface of a substrate on which a physiologically active substance has been immobilized, and signals generated during the supply of the solution containing no test substances can be obtained as baseline values. The physiologically active substance immobilized on the surface of the substrate is eliminated as time passes. As a result, the obtained baseline values are also likely to decrease as time passes in many cases. Using the thus obtained baseline values, a calibration curve can be obtained.

In general, such a baseline is measured as a standard for a change in signal. However, when such a baseline itself continuously changes and a change in baseline is too large with respect to a change in signal to be detected, even if a temporal difference is small, the effect of such a temporal difference cannot be prevented by simple subtraction of such baseline, and thus a precise signal cannot be detected. The present invention is characterized in that the effect of a temporal difference generated during the measurement of a baseline and during the measurement of a test substance can be calibrated based on the tendency of a change obtained by a repeated measurement of baseline values.

The term "a single type of solution" used for obtaining baseline values means a solution having a same composition that contains no test substances. Solutions prepared by any methods may be used herein, as long as they have a same composition. A method of repeatedly using a solution placed in a hermetically-sealed vessel that prevents a change in composition caused by evaporation, contamination, etc. is preferable. The term "a single type of solution" is used herein to mean a solution having a composition including an error in salt concentration of less than 1% and errors in the concentrations of other solutes of less then 10%.

A calibration curve is preferably a curve obtained by approximating baseline values by the least square method. The least square method is a method described in detail in the publication *"Pasokon ni yoru Data Bunseki* (Data Analyses by Personal Computers)" (Masakazu Onishi, Kenpakusha) Chapter 4, 1. *Keiko Hendo no Bunseki* (Analyses of Changes in Trend). This is a method for obtaining a regression coefficient such that the sum of squares of deviation from the regression formula becomes the minimum.

As the calibration curve of the present invention, any one of a first exponential curve (a linear approximation), a second exponential curve, a third exponential curve and an exponential approximation is preferable used. These calibration curves can be easily obtained using the function of Microsoft Excel.

Baseline values used for obtaining the calibration curve of the present invention are values obtained by repeatedly measuring the surface of a sensor with a single type of solution. A calibration curve is an approximation formula. Larger the number of baseline values, a higher precision that can be obtained. Thus, the number of baseline values is preferably large. The number of such baseline values is preferably between 4 and 1000, and more preferably between 10 and 500.

In the present invention, the measurement value of a test substance is calibrated by a calibration curve. In order to calibrate the measurement value of a test substance by a calibration curve, a difference from the calibration curve may be subtracted from the measurement value of the test substance, so as to obtain calibration data.

The substrate for immobilizing a physiologically active substance which is used in the present invention is preferably a metal surface or a metal film. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above carrier, an interstitial layer consisting of chrome or the like may be provided between the carrier and a metal layer.

The film thickness of a metal film is not limited. When the metal film is used for a surface plasmon resonance biosensor, the thickness is preferably between 0.1 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, and nonelectrolytic plating method.

The metal film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate. When a substrate of the present invention is used for a surface plasmon resonance biosensor, examples of a substrate may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used.

The substrate in the present invention is preferably a substrate which was coated with a hydrophilic polymer having a reactive group. The hydrophilic polymer having a reactive group can be bound to the above-described metal surface or metal film directly or via an intermediate layer.

Examples of a hydrophilic polymer compound include polysaccharides (e.g., agarose, dextran, carrageenan, alginic acid, starch, and cellulose) and synthetic polymer compounds (e.g., polyvinyl alcohol). In the present invention, a polysaccharide is preferably used, and dextran is most preferably used.

In the present invention, preferably, a hydrophilic polymer with an average molecular weight of 10,000 to 2,000,000 can be used. Preferably, a hydrophilic polymer with an average molecular weight of 20,000 to 2,000,000, further preferably 30,000 to 1,000,000, and most preferably 200,000 to 800,000 can be used.

A hydrophilic polymer which is immobilized on the surface of a sensor preferably has a thickness between 1 and 300 nm in an aqueous solution. If the film thickness is too small, the quantities of physiologically active substances immobilized are reduced. In addition, since a hydration layer on the sensor surface becomes thin, physiologically active substances denature by themselves, and it thereby becomes difficult to detect the interaction of such physiologically active substances with test substances. On the other hand, if the film thickness is too large, it impairs dispersions of test substances in the film. Moreover, in particular, when such interaction is detected from the side opposite to the surface of a sensor substrate on which a hydrophilic polymer is immobilized, the distance between a detection surface and an interaction-forming portion is increased, resulting in a decrease in detection sensitivity. The film thickness of a hydrophilic polymer in an aqueous solution can be evaluated by AFM, ellipsometry measurement, etc.

A polyhydroxy compound that is an example of the hydrophilic polymer can be carboxylated by reacting the compound with bromoacetic acid under basic conditions, for example. Through control of the reaction conditions, a given proportion of hydroxy groups contained in the polyhydroxy compound in its initial state can be carboxylated. In the present invention, 1% to 90% of hydroxy groups can be carboxylated, for example. In addition, the degree of carboxylation (% carboxylation) on a surface coated with any polyhydroxy polymer compound can be calculated by the following method, for example. The film surface is subjected to gas-phase modification at 50°C for 16 hours using di-tert-butyl carbodiimide/pyridine catalyst and trifluoroethanol. A fluorine amount derived from trifluoroethanol is measured via ESCA (electron spectroscopy for chemical analysis). The ratio of the fluorine amount to an oxygen amount on the film surface (hereinafter, referred to as an F/O value) is then calculated. The theoretical F/O value when all the hydroxy groups are carboxylated is determined to represent 100% carboxylation. The F/O value resulting when carboxylation is performed under arbitrary conditions is measured and then the degree of carboxylation (% carboxylation) at this time can be calculated.

An approach known in the art, for example, a method comprising performing activation with 1-(3-Dimethylaminopropyl)-3 ethylcarbodiimide (EDC) as water-soluble carbodiimide and N-Hydroxysuccinimide (NHS), or a method comprising performing activation with EDC alone, can be used preferably as a method of activating the polymer containing a carboxyl group. The polymer containing the carboxyl group activated by this approach can be reacted with the substrate having an amino group to thereby produce the biosensor of the present invention.

The reaction group introduced into a polymer as a group for immobilizing a physiologically active substance may be a carboxyl group or an amino group. Also, a state is also possible in which a physiologically active substance such as biotin-binding protein (such as avidin, streptavidin, or neutravidin), Protein A, Protein G, an antigen, or an antibody (for example, a known tag antibody such as an anti-GST antibody) is previously immobilized. A physiologically active substance having a membrane structure such as a lipid, can be immobilized by using an immobilization layer of a polymer to which alkane is introduced. According to an application purpose, the length of a polymer chain, the thickness of a polymer, the density of a polymer, or the amount of a reaction group to be introduced into a polymer is controlled. As a result, it is possible to be applied to various types of protein. Furthermore, a His-tag ligand or the like can be immobilized through a metal chelate by introducing NTA (nitrilotriacetic acid) or the like as an immobilization group into a polymer.

In the present invention, a hydrophilic polymer having reactive groups can be bound to a metal surface or a metal film directly or indirectly via an intermediate layer. As such "intermediate layer" used herein, a layer comprising a hydrophobic polymer compound or a self-assembled membrane can be used, for example. Hereinafter, the hydrophobic polymer compound and the self-assembled membrane will be explained.

The hydrophobic polymer compound is a polymer compound lacking water-absorbing properties and having solubility (25°C) in water of 10% or less, more preferably 1% or less, and most preferably, 0.1% or less.

Hydrophobic monomers that form such hydrophobic polymer compound can be arbitrarily selected from among vinyl esters, acrylic acid esters, methacrylic acid esters, olefins, styrenes, crotonic acid esters, itaconic acid diesters, maleic acid diesters, fumaric acid diesters, allyl compounds, vinyl ethers, vinyl ketones, and the like. Such hydrophobic polymer compound may be a homopolymer comprising one type of monomer, or a copolymer comprising two or more types of monomer.

Examples of such hydrophobic polymer compound that is preferably used in the present invention include polystyrene, polyethylene, polypropylene, polyethylene terephthalate, polyvinylchloride, polymethyl methacrylate, polyester, and nylon.

A carrier can be coated with such hydrophobic polymer compound by a conventional method such as spin coating, air-knife coating, bar coating, blade coating, slide coating, or curtain coating. Coating can also be performed by a spray method, an evaporation method, a cast method, a dipping method, or the like.

The coating thickness of the hydrophobic polymer compound is not particularly limited and is preferably 0.1 nm to 500 nm, and particularly preferably 1 nm to 300 nm.

Next, the self-assembled membrane will be explained. Sulfur compounds such as thiol and disulfides are spontaneously adsorbed onto a noble metal (e.g., gold) substrate, so that a monomolecular-sized ultra-thin film can be produced. A cluster of such sulfur compounds has a sequence depending on the crystal lattice of a substrate or the molecular structure of the adsorbed molecules. Hence, the thus provided membrane is referred to as a self-assembled membrane. Specifically, in the present invention, a hydrophilic polymer can be adhered to a metal film via an organic molecule X¹-R¹-Y¹. The organic molecule X¹-R¹-Y¹ will be described in detail.

X¹ is a group having binding affinity for a metal film. Specifically, asymmetric or symmetric sulfide (-SSR¹¹Y¹¹, -SSR¹Y¹), sulfide (-SR¹¹Y¹¹, -SR¹Y¹), diselenide (-SeSeR¹¹Y¹¹, -SeSeR¹Y¹), selenide (SeR¹¹Y¹¹, -SeR¹Y¹), thiol (-SH), nitrile (-CN), isonitrile, nitro (-NO₂) selenol (-SeH), a trivalent phosphorus compound, isothiocyanate, xanthate, thiocarbamate, phosphine, thio acid, or dithioic acid (-COSH, -CSSH) is preferably used.

R¹ (and R¹¹) is occasionally interrupted via hetero atoms, is preferably linear (unbranched) because of appropriately dense packing, and is occasionally a hydrocarbon chain containing double and/or triple bonds. The length of the chain is preferably 10 or more atoms. The carbon chain can be occasionally perfluorinated.

Y¹ and Y¹¹ are groups for binding with a polyhydroxy polymer compound. Y¹ and Y¹¹ are preferably the same and are capable of binding directly with such polyhydroxy polymer compound or binding with the same after activation. Specifically, a hydroxyl, carboxyl, amino, aldehyde, hydrazide, carbonyl, epoxy, or vinyl group can be used, for example.

In the present invention, 7-carboxy-1-heptanethiol, 10-carboxy-1-decanethiol, 4,4'-dithiodibutyric acid, 11-hydroxy-1-undecanethiol, 11-amino-1-undecanethiol, and the like can be used as self-assembled compounds, for example.

In the present invention, a metal film is covered with an organic layer having an amino group, and then the organic layer is reacted with a polymer having an activated carboxyl group. Thus, a hydrogel capable of immobilizing a physiologically active substance can be produced.

In the present invention, a known method can be used as a method for coating a metal film with an organic layer having an amino group. In view of simple operation, a method for coating with the use of a self-assembled membrane (SAMs) is preferred. A method for coating a metal film with the use of a self-assembled membrane (SAMs) has been actively developed by Professor Whitesides et al. (Harvard University). Details of the method are reported in, for example, Chemical Review, 105, 1103-1169 (2005). When gold is used as a metal, an orientational self-assembled monomolecular film is formed with the use of an alkanethiol derivative represented by the following formula A-1 (in the formula A-1, n represents an integer from 3 to 20, and X represents a functional group) as an organic layer-forming compound based on the van der Waals force between an Au-S bond and an alkyl chain. A self-assembled membrane is formed by a very simple method, wherein a gold substrate is immersed in a solution of an alkanethiol derivative. A self-assembled membrane is formed with the use of a compound represented by the following formula A-1 where X is NH₂, so that it becomes possible to coat a gold surface with an organic layer comprising an amino group:

**HS(CH₂)ₙX** **A-1**

An alkanethiol having an amino group at the end may be a compound comprising a thiol group and an amino group linked via an alkyl chain (formula A-2) (in the formula A-2, n represents an integer of 3 to 20), or may be a compound obtained by reaction between alkanethiol having a carboxyl group at the end (formula A-3 or A-4) (in the formula A-3, n represents an integer of 3 to 20, and in the formula A-4, n each independently represents an integer of 1 to 20) and a large excess of hydrazide or diamine. The reaction between alkanethiol having a carboxyl group at the end and a large excess of hydrazide or diamine may be performed in a solution state. Alternatively, the alkanethiol having a carboxyl group at the end may be bound to the substrate surface and then reacted with a large excess of hydrazide or diamine.

**HS(CH₂)ₙNH₂** **A-2**

**HS(CH₂)ₙCOOH** **A-3**

**HS(CH₂)ₙ(OCH₂CH₂)ₙOCH₂COOH** **A-4**

The repeating number of alkyl group of the formulas A-2 to A-4 is preferably 3 to 20, more preferably 3 to 16, and most preferably 4 to 8. If the alkyl chain is short, formation of self-assembled membrane becomes difficult, and if the alkyl chain is long, water solubility decreases and the handling becomes difficult.

Any compound may be used as the diamine used in the present invention. An aqueous diamine is preferable for use in the biosensor surface. Specific examples of the aqueous diamine may include aliphatic diamine such as ethylenediamine, tetraethylenediamine, octamethylenediamine, decamethylenediamine, piperazine, triethylenediamine, diethylenetriamine, triethylenetetraamine, dihexamethylenetriamine, and 1.4-diaminocyclohexane, and aromatic diamine such as paraphenylenediamine, metaphenylenediamine, paraxylylenediamine, metaxylylenediamine, 4,4'-diaminobiphenyl, 4,4'-diaminodiphenylmethane, 4,4'-diaminodiphenylketone, and 4,4'-diaminodiphenylsulfonic acid. From the viewpoint of increasing the hydrophilicity of the biosensor surface, a compound comprising two amino groups linked via an ethylene glycol unit (formula A-5) may also be used. The diamine used in the present invention is preferably ethylenediamine or the compound represented by the formula A-5 (in the formula A-5, n and m each independently represent an integer of 1 to 20), more preferably ethylenediamine or 1,2-bis(aminoethoxy)ethane (represented by the formula A-5 wherein n=2 and m=1).

**H₂N(CH₂)ₙ(OCH₂CH₂)ₘO(CH₂) ₙN^{H}₂** **A-5**

The alkanethiol having an amino group may form a self-assembled membrane by itself or may form a self-assembled membrane by mixing it with another alkanethiol. It is preferred for use in the biosensor surface that a compound capable of suppressing the nonspecific adsorption of a physiologically active substance should be used as the another alkanethiol. The aforementioned Professor Whitesides et al. have investigated in detail self-assembled membrane capable of suppressing the nonspecific adsorption of a physiologically active substance and have reported that a self-assembled membrane formed from alkanethiol having a hydrophilic group is effective for suppressing nonspecific adsorption (Langmuir, 17, 2841-2850, 5605-5620, and 6336-6343 (2001)). In the present invention, any of compounds described in the aforementioned papers may be used preferably as the alkanethiol that forms a mixed monolayer with an alkanethiol having an amino group. In terms of excellent ability to suppress nonspecific adsorption and ease of acquisition, it is preferred that alkanethiol having a hydroxyl group (formula A-6) or alkanethiol having an ethylene glycol unit (formula A-7) (in the formula A-6, n represents an integer of 3 to 20, and in the formula A-7, n and m each independently represent an integer of 1 to 20) should be used as the alkanethiol that forms a mixed monolayer with an alkanethiol having an amino group.

**HS(CH₂)ₙOH HS(CH₂)ₙ(OCH₂CH₂)ₘOH A-6** **A-7**

When alkane thiol having an amino group is mixed with another alkane thiol to form a self-assembled membrane, the repeating number of alkyl group of the formulas A-2 to A-4 is preferably 4 to 20, more preferably 4 to 16, and most preferably 4 to 10. Further, the repeating number of alkyl group of the formulas A-6 and A-7 is preferably 3 to 16, more preferably 3 to 12, and most preferably 3 to 8.

In the present invention, it is possible to mix alkanethiol having an amino group and alkanethiol having a hydrophilic group at an arbitrary ratio. However, when the content of alkanethiol having an amino group is low, the amount of actively esterified carboxyl group-containing polymer to be bound decreases. When the content of alkanethiol having a hydrophilic group is low, the capacity for suppression of nonspecific adsorption is reduced. Thus, the mixing ratio of alkanethiol having an amino group to alkanethiol having a hydrophilic group is preferably 1:1 to 1:1,000,000, more preferably 1:4 to 1:10,000, and further preferably 1:10 to 1:1,000. In view of reduction of steric hindrance upon a reaction with an actively esterified carboxyl group-containing polymer, the molecular length of alkanethiol having an amino group is preferably longer than that of alkanethiol having a hydrophilic group.

As alkanethiol used for the present invention, compounds synthesized based on Abstract, Curr. Org. Chem., 8, 1763-1797 (2004) (Professor Grzybowski, Northwestern University) and references cited therein or a commercially available compound may be used. It is possible to purchase such compounds from Dojindo Laboratories, Aldrich, SensoPath Technologies, Frontier Scientific Inc., and the like. In the present invention, disulfide compounds that are oxidation products of alkanethiol can be used in the same manner as alkanethiol.

A physiologically active substance immobilized on the substrate in the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant *Staphylococcus aureus,* antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55, 111 and 157 among enteropathogenic *Escherichia coli.*

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant *Staphylococcus aureus,* antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as *Escherichia coli* can be used.

As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.

As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor.

Examples of an immunoglobulin-binding protein used herein may include protein A, protein G, and a rheumatoid factor (RF).

As a sugar-binding protein, for example, lectin is used.

Examples of fatty acid or fatty acid ester may include stearic acid, arachidic acid, behenic acid, ethyl stearate, ethyl arachidate, and ethyl behenate.

When the physiologically active substance is a protein such as an antibody or an enzyme, or a nucleic acid, the immobilization can be carried out by covalently binding the physiologically active substance to a reactive group on a substrate with the use of amino group, thiol group or the like of the physiologically active substance.

As the test substance, for example, a sample containing a substance which interacts with the above-mentioned physiologically active substances can be used.

With regard to the concentration of a solution (an applied solution) that contains physiologically active substance, it is preferable that the concentration of physiologically active substance immobilized on a substrate surface be high. The aforementioned concentration is preferably between 0.1 mg/ml and 10 mg/ml, and more preferably between 1 mg/ml and 10 mg/ml, although it depends on the type of physiologically active substance.

In the process of drying a solution that contains physiologically active substance, the physiologically active substance tend to be precipitated from the peripheral portion of the applied solution, or from a portion in which the liquid remains immediately before the applied solution is dried. Thereby, the quantities of physiologically active substance immobilized on the substrate surface vary. This is not preferable. In order to uniform the quantities of physiologically active substance immobilized on the substrate surface, it is preferable that the viscosity of the applied solution be set at high, so far as it does not inhibit the binding of the physiologically active substance to the substrate surface. By setting the viscosity of the applied solution at high, the movement of the physiologically active substance contained in the applied solution in the horizontal direction towards the substrate surface can be suppressed during the drying process. As a result, variations in the quantities of physiologically active substance immobilized can be suppressed. The viscosity of the applied solution is preferably maintained at 0.9 cP or more during the drying process.

The term "drying process" is used in the present invention to mean natural drying whereby after application of a solution that contains a physiologically active substances, the solution is left at rest, or an intentional drying process whereby the speed of drying the aforementioned solution is increased by heating or air-blowing. An increase in the drying rate of the solution that contains the physiologically active substance (coating solution) is also effective for suppressing variation in the amount of the physiologically active substance immobilized. The drying rate is increased, and the drying process is sufficiently rapidly completed with respect to the movement of the physiologically active substance in the horizontal direction. Thereby, the drying process is completed before the physiologically active substance substantially moves, so that the variation can be suppressed. When such a coating solution contains water, a high drying rate can be obtained by drying the coating solution in an environment wherein a difference between a dry-bulb temperature and a wet-bulb temperature is great. The coating solution is dried in an environment wherein a temperature difference between the dry-bulb temperature and the wet-bulb temperature is preferably 7°C or greater, and more preferably 10°C or greater, further preferably 13.5°C or greater. In addition, considering the production process, the drying time is preferably 10 minutes or shorter, more preferably 5 minutes or shorter, and particularly preferably 1 minute or shorter.

An example of a method of applying a solution that contains physiologically active substance is a method particularly using a dispenser that quantitatively discharges a solution to be applied. The discharge port of the dispenser is moved on a substrate at a certain speed at certain intervals, so that the solution can be uniformly applied at any given sites on the substrate. When the solution is applied using a dispenser, the interval between the substrate and the discharge port is extremely narrowed, and the thickness of the applied solution is reduced, so that the thickness of physiologically active substance can be uniformed. Further, the drying speed can also be increased. Thus, the use of such a dispenser is preferable. Another preferred method of applying a solution that contains physiologically active substance is spin coating. This method is particularly preferably applied when the thickness of the applied film is reduced. In this method, since the drying process is carried out after a solution having a uniformed thickness has been formed, it is preferable to prevent evaporation of the solution during rotation of a spin coater. If a method of placing a substrate in a hermetically sealed vessel or the like during rotation is applied to maintain the concentration of a solvent existing around the substrate at high, the drying speed can be controlled before and after formation, of a thin film during rotation. Thus, this method is particularly preferable. It is preferred that drying is carried out under a condition where temperature and humidity are kept constant, after this coating process.

When the interaction of physiologically active substance with test substances is detected, variations in the quantities of physiologically active substance immobilized on the sensor surface cause an error in quantitative and kinetic evaluation of such an interaction. In order to keep such an error to a minimum, the quantities of physiologically active substance immobilized are preferably uniformed. A CV value (coefficient variation) (standard deviation/mean value), which indicates variations in the quantities of physiologically active substance immobilized on the surface of a substrate used in detection of an interaction, is preferably 15% or less, and more preferably 10% or less. Such a CV value can be calculated based on the quantities of physiologically active substance immobilized on at least two sites, preferably 10 or more sites, and more preferably 100 or more sites on the substrate surface. Uniformity can be evaluated by quantifying the quantities of substances existing on a sensor substrate before and after immobilization of physiologically active substance. However, such uniformity can also be evaluated by fluorescently-labeling substances that have been known to bind to physiologically active substance, immobilizing such fluorescently-labeled substances on a sensor substrate, and then measuring fluorescence intensity using a fluorescence microscope or the like. Moreover, it is also possible to quantify physiologically active substance using an SPR imager, an ellipsometer, TOF-SIMS, an ATR-IR apparatus, etc.

Generally, physiologically active substance such as protein maintain its three-dimensional structure by coordination of water molecules in a solution, but when it is dried, physiologically active substance cannot maintain its three-dimensional structure and is denatured. Further, physiologically active substance is contained in a hydrophilic polymer compound on a surface of substrate, physiologically active substance aggregate by drying, and aggregates are produced. A compound having a residue capable of forming hydrogen bond (hereinafter referred to as Compound S) of the present invention can be used for the purpose of suppressing denature of physiologically active substance by maintaining the three-dimensional structure in place of water or suppressing the aggregation by steric effect by covering the physiologically active substance.

In the present invention, the Compound S having a residue capable of forming hydrogen bond is preferably added as a aqueous solution to a layer on substrate where physiologically active substance was immobilized. The Compound S can be added by coating a mixed solution of the Compound S and physiologically active substance on a surface of substrate, or by immobilizing physiologically active substances on a surface of substrate and then over-coating the polymer. When a mixed solution of the Compound S and physiologically active substance is coated, fluctuation of the amount of immobilized physiologically active substances can be reduced. Preferably, an aqueous solution of the Compound S can be added to substrate in a state of thin film. A method for forming thin film on substrate may be any known method. Examples thereof include extrusion coating, curtain coating, casting, screen printing, spin coating, spray coating, slidebead coating, slit and spin coating, slit coating, die coating, dip coating, knife coating, blade coating, flow coating, roll coating, wire-bar coating, and transferring printing. In the present invention, spray coating or spin coating is preferably used, and spin coating is more preferably used as a method for forming a thin film on substrate, since a coated film having a controlled film thickness can be easily prepared.

The concentration of the applied solution of Compound S is not particularly limited, as long as it does not cause a problem regarding permeation into a layer that contains physiologically active substances. The aforementioned concentration is preferably between 0.1% by weight and 5% by weight. In addition, in terms of applicability and regulation of pH, a surfactant, a buffer, an organic solvent, a salt may also be added to the applied solution.

The compound S having a residue capable of forming hydrogen bond is preferably a compound which is non-volatile under normal pressure at normal temperature. The average molecular weight of the compound is preferably 350 to 5,000,000, more preferably 1,200 to 2,000,000, most preferably 1,200 to 70,000. The compound S having a hydroxyl group in molecule is preferably saccharide. The saccharide may be monosaccharide or .polysaccharide. In case of n-saccharide, n is preferably 4 to 1,200, and n is more preferably 20 to 600.

If the mean molecular weight of compound S is too low, the compound is crystallized on the surface of a substrate. This causes disruption of a hydrophilic polymer layer, on which physiologically active substances are immobilized, and disruption of the three-dimensional structure of the physiologically active substances. In contrast, if the mean molecular weight of compound S is too high, it causes problems such that it impairs immobilization of physiologically active substances on a substrate, that a layer that contains physiologically active substances cannot be impregnated with compound S, and that layer separation occurs.

For the purpose of suppressing degradation of physiologically active substances immobilized on a substrate, the aforementioned compound S having a residue capable of forming hydrogen bond preferably has a dextran skeleton or a polyethylene oxide skeleton. The type of a substituent used is not limited, as long as the object of the present invention can be achieved. Moreover, for the purpose of suppressing degradation of physiologically active substances immobilized on a substrate, a nonionic compound having no dissociable groups is preferably used as compound S. Furthermore, the aforementioned compound S having a residue capable of forming a hydrogen bond preferably has high affinity for water molecules. A distribution coefficient LogP value between water and n-octanol is preferably 1 or greater. Such LogP value can be measured by the method described in Japanese Industrial Standard (JIS), Z7260-107 (2000), "Measurement of distribution coefficient (1-octanol/water) - Shaking method," etc.

Specific examples of compound S having a residue capable of forming hydrogen bond include: compounds consisting of two or more types of residues selected from polyalcohols such as polyvinyl alcohol, proteins such as collagen, gelatin, or albumin, polysaccharides such as hyaluronic acid, chitin, chitosan, starch, cellulose, alginic acid, or dextran, polyethers including polyethyleneoxy-polypropylene oxide condensates such as polyethylene glycol, polyethylene oxide, polypropylene glycol, polypropylene oxide, or Pluronic, Tween 20, Tween 40, Tween 60, Tween 80, etc.; and derivatives and polymers of such compounds. Of these, polysaccharides and polyethers are preferable, and polysaccharides are more preferable. Specifically, dextran, cellulose, Tween 20, Tween 40, Tween 60, and Tween 80 are preferably used. Further, a nonvolatile monomer and a nonvolatile water-soluble oligomer described in JP Patent Publication (Kokai) No. 2006-170832 A can also be used. Examples of such a nonvolatile monomer used herein may include: tetrose, pentose, heptose and hextose, wherein a hydroxyl group may be protected by a protecting group, and their glycoside; methyl glucoside; and cyclitols, wherein a hydroxyl group may be protected by a protecting group. Moreover, examples of such a nonvolatile water-soluble oligomer include: an oligomer represented by general formula (1) (-[CH2-CH(CONH2)-]n-), general formula (2) (-[CH2-CH2-O-]n-), or general formula (3) (-[CH2-CH(OH)-]n-) (wherein, in general formulas (1) to (3), n represents an integer between 10 and 200); and an oligosaccharide having an n number of sugars (2 ≤ n ≤ 10), wherein a hydroxyl group may be protected by a protecting group. Furthermore, sugars described in US 2003/0175827 and DE 20306476A1, such as trehalose, sucrose, maltose, lactose, xylitol, fructose, mannitol, glucose, xylol, maltodextran, saccharose, or polyvinylpyrrolidone may also be used. It is preferable that such compound S be substantially identical to the basic skeleton of a hydrophilic polymer used in the present invention. The term "basic skeleton" is used herein to mean a ring structure of sugar, for example. Although the type of a functional group or length differs, if such a ring structure is identical, it is considered that the basic skeleton is substantially identical.

The measurement method of the present invention can be performed by using commercially available measurement apparatus. For example, in the case of SPR analysis, Biacore3000, 2000, 1000, A100, T100, S51, X, J (GE Healthcare (Biacore)), SPR-670 and SPR-MACS (Moritex corporation), MulriSPRinter (TOYOBO), and SPR Imager (GWC Technologies) can be used. Further, Epic System (Corning Inc.), AnaLight (Farfield Scientific), AFFINIX (ULVAC Co. (Initium) and the like can be used. Further, the device described on paragraphs 0025-0062 of Japanese Patent Publication (Kokai) No.2006-194624 can also be used.

The measurement method of the present invention can be performed by using a biosensor. The biosensor used in the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance is immobilized on a substrate to be used as a molecule-recognizing substance, so that the corresponding substance can be selectively measured.

In the present invention, an interaction between a physiologically active substance and a test substance is preferably detected and/or assayed by a nonelectrochemical method. Examples of nonelectrochemical methods include surface plasmon resonance (SPR) assay, quartz crystal microbalance (QCM) oscillation assay, and assay techniques involving the use of functionalized surfaces of particles, ranging from gold colloid particles to ultrafine particles.

In a preferred embodiment of the present invention, the biosensor can be used as a biosensor for surface plasmon resonance which is characterized in that it comprises a metal film placed on a transparent substrate.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.

A device using a system known as the Kretschmann configuration is an example of a surface plasmon measurement device for analyzing the properties of a substance to be measured using a phenomenon whereby a surface plasmon is excited with a lightwave (for example, paragraph 0011 of Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light. Accordingly, it is necessary to set the light beam in advance such that it enters as p-polarized light.

If the wave number of a surface plasmon is determined from an incident angle causing the attenuated total reflection (ATR), that is, an attenuated total reflection angle (θSP), the dielectric constant of a measured substance can be determined. As described on paragraphs 0025-0037 of Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases.

In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.

In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface. Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.

In this leaking mode measurement device also, the above-described array-form light-detecting means can be used to detect the position of a dark line generated in a reflected light due to attenuated total reflection. In addition, the above-described differentiating means can also be applied in combination with the above means.

The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed.

If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Publication (Kokai) No. 2003-172694 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.

Moreover, Japanese Patent Laid-Open No. 2001-330560 describes a measurement device using attenuated total reflection, which involves successively measuring multiple measurement chips mounted on a turntable or the like, so as to measure many samples in a short time.

The biosensor of the present invention can be used as a biosensor, which has a waveguide structure on the surface of a carrier, for example, and which detects refractive index changes using such a waveguide. In this case, the waveguide structure on the carrier surface has a diffraction grating, and in some cases, an additional layer. This waveguide structure is a planar waveguide body comprising a thin dielectric layer. Light gathered to the waveguide body form is introduced into such a thin layer by total internal reflection. The transmission velocity of the thus introduced light wave (hereinafter referred to as "mode") is indicated as a C/N value. Herein, C indicates the velocity of light in a vacuum, and N indicates an effective refractive index of the mode introduced into the waveguide body. Such an effective refractive index N is determined based on the structure of the waveguide body on one face, and is determined based on the refractive index of a medium adjacent to the thin waveguide layer on the other face. Conduction of a light wave is carried out not only in a thin planar layer, but also by another waveguide structure, and in particular, by a stripped waveguide body. In such a case, the waveguide structure is processed into the shape of a stripped film. It is an important factor for a biosensor that changes in effective refractive indexes N are generated as a result of changes in the medium adjacent to the waveguide layer, and changes in the refractive index and thickness of the waveguide layer itself or an additional layer adjacent to the waveguide layer.

The structure of a biosensor of this system is described in page 4, line 48 to page 14, line 15, and Figures 1 to 8 of JP Patent Publication (Kokoku) No. 6-27703 B (1994), and column 6, line 31 to column 7, line 47, and Figures 9A and 9B of US Patent No. 6,829,073.

For example, in one embodiment, there is a structure whereby a waveguide layer comprising a planar thin layer is established on a substrate (e.g. Pyrex (registered trademark) glass). A waveguide layer and a substrate form together a so-called waveguide body. Such a waveguide layer can be a multilayer laminated body such as an oxide layer (SiO₂, SnO₂ Ta₂O₅, TiO₂, TiO₂-SiO₂, HfO₂, ZrO₂, Al₂O₃, Si₃N₄, HfON, SiON, scandium oxide, or a mixture thereof) or a plastic layer (e.g. polystyrene, polyethylene, polycarbonate, etc.). For transmission of light into a waveguide layer as a result of total internal reflection, the refractive index of the waveguide layer must be greater than that of the adjacent medium (for example, a substrate, or an additional layer as described later). A diffraction grating is disposed on the surface of the waveguide layer or in the bosom thereof, which faces to a substrate or a measured substance. Such a diffraction grating can be formed in a carrier according to embossing, holography, or other methods. Subsequently, the upper surface of the diffraction grating is coated with a thin waveguide film having a higher refractive index. The diffraction grating has the functions to focus rays of light incident on the waveguide layer, to discharge the mode already introduced into the waveguide layer, or to transmit a portion of the mode in the travel direction and reflect a portion thereof. The grating area of the waveguide layer is covered with an additional layer. Such an additional layer can be a multilayer film, as necessary. This additional layer is able to have the function to carry out selective detection of a substance contained in a measured substance. In a preferred embodiment, a layer having a detection function can be established on the outermost surface of such an additional layer. As such a layer having a detection function, a layer capable of immobilizing physiologically active substances can be used.

In another embodiment, it is also possible to adopt a structure whereby an array of diffraction grating waveguides is incorporated into wells of a microplate (JP Patent Publication (Kohyo) No. 2007-501432 A). That is to say, if such diffraction grating waveguides are aligned in the form of an array at the bottoms of wells of a microplate, the screening of a drug or chemical substance can be carried out at a high throughput.

In order to detect physiologically active substances existing on the upper layer (detection area) of a diffraction grating waveguide, the diffraction grating waveguide detects incident light and reflected light, so as to detect changes in refractive properties. For this purpose, one or more light sources (e.g. laser or diode) and one or more detectors (e.g. a spectrometer, a CCD camera, or other light detectors) can be used. As a method of measuring changes in refractive indexes, there are two different operational modes, namely, spectroscopy and an angle method. In spectroscopy, broadband beam used as incident light is transmitted to a diffraction grating waveguide, and reflected light is gathered, followed by a measurement with a spectrometer, for example. By observing the spectrum position of a resonant wavelength (peak), changes in refractive indexes on the surface of the diffraction grating waveguide or a periphery thereof, namely, a bond can be measured. On the other hand, in an angle method, light of a nominally single wavelength is gathered such that it generates a certain range of irradiation angle, and it is directed into the diffraction grating waveguide. The reflected light is measured with a CCD camera or other types of light detectors. By measuring the position of a resonance angle reflected by the diffraction grating wavelength, changes in refractive indexes on the surface of the diffraction grating waveguide or a periphery thereof, namely, a bond can be measured.

The present invention will be further specifically described in the following examples. However, the examples are not intended to limit the scope of the present invention.

### EXAMPLES

### Example 1

A hydrogel film capable of immobilizing a protein thereon was produced using an SAM compound with high water-solubility. Thereafter, the amount of a protein immobilized and non-specific adsorptive performance were evaluated.

### (1) Preparation of substrate

An aqueous solution of 1 mM 6-Amino-1-octanethiol, hydrochloride (manufactured by Dojindo Laboratories) was prepared. This solution was designated as Solution A.

Next, a gold thin film was produced on the upper surface of a plastic prism obtained by injection-molding ZEONEX (manufactured by Zeon Corp) according to a method described below. The prism was secured to a substrate holder of a sputtering apparatus, and a vacuum (base pressure of 1 × 10⁻³ Pa or less) was drawn therein. Thereafter, Ar gas was introduced (1 Pa) into the apparatus, and RF power (0.5 kW) was applied to the substrate holder for approximately 9 minutes with the substrate holder rotated (20 rpm), so as to plasma-treat the prism surface. Next, Ar gas was stopped, and a vacuum was drawn therein. Ar gas was introduced (0.5 Pa) again into the apparatus, and DC power (0.2 kW) was applied to a 8-inch Cr target for approximately 30 seconds with the substrate holder rotated (10 to 40 rpm), so as to form a 2-nm Cr thin film thereon. Subsequently, Ar gas was stopped, and a vacuum was drawn again. Ar gas was introduced (0.5 Pa) again into the apparatus, and DC power (1 kW) was applied to a 8-inch Au target for approximately 50 seconds with the substrate holder rotated (20 rpm), so as to form an approximately 50-nm Au thin film thereon.

The thus obtained sensor stick B, on which the Au thin film had been formed, was immersed in the Solution A at 40°C for 1 hour and was then washed five times with ultrapure water.

### (2) Active esterification of CMD (carboxymethyl dextran)

After dissolution of 10 g of a solution of 1% by weight of CMD (manufactured by Meito Sangyo; molecular weight of 1,000,000; substitution degree of 0.65), 10 ml of a mixed aqueous solution of 0.02 M EDC (1-Ethyl-3-[3-Dimethylaminopropyl]carbodiimide Hydrochloride) and 87.5 mM HOBt (1-Hydroxybenzotriazole) was added thereto, followed by stirring at room temperature for 1 minute. Thereafter, the reaction solution was left at rest for 1 hour.

### (3) Binding reaction of CMD to substrate

Onto the substrate prepared in (1) above, 1ml of the active esterified CMD solution prepared in (2) above was added dropwise and was then spin-coated at 1000 rpm for 45 seconds to thereby form a thin film of the actively esterified carboxymethyl dextran on the substrate having an amino group. After reaction at room temperature for 15 minutes, the substrate was immersed in 1N NaOH for 30 minutes, and was then washed 5 times with ultrapure water to obtain a CMD-immobilized substrate.

### (4) Binding reaction of NeutrAvidin to CMD-immobilized substrate

The substrate prepared in (3) above was equipped in a surface plasmon resonance apparatus. A mixed aqueous solution of 0.2 M EDC and 0.05 M NHS (N-Hydroxysuccinimide) was placed on the substrate, and it was then left at rest for 7 minutes. Thereafter, the surface of the substrate was washed with Acetate5.0 (manufactured by BIACORE), followed by air-blowing. Thereafter, an Acetate5.0 solution of 0.15 mg/ml NeutrAvidin (manufactured by PIERCE) was placed thereon, and it was then left at rest for 15 minutes. Thereafter, the surface of the substrate was washed with a 1 M Ethanolamine aqueous solution (adjusted to pH 8.5), and it was then left at rest for 7 minutes. Thereafter, the surface was immersed in a 10 mM NaOH aqueous solution for 1 minute and was then washed with 1 × PBS (pH 7.4). This operation was repeated once more, so as to obtain a substrate on which NeutrAvidin had been immobilized.

The signals obtained before and after immobilization of NeutrAvidin were measured using a surface plasmon resonance apparatus. As a result, the amount of NeutrAvidin immobilized was found to be 11000 RV on average. Herein, a difference of resonance angle per % of DMSO is indicated as 1500 RV.

### (5) Biotinylation of CA

0.5 ml of 1 × PBS (pH 7.4) solution of 0.045 mg/ml SNHS-LC-LC-Biotin (manufactured by PIERCE) was added at 4°C to a 1.5-ml microtube that contained 0.5 ml of 1 × PBS (pH 7.4) solution of 2 mg/ml Carbonic Anhydrase (manufactured by SIGMA; hereinafter abbreviated as CA). The obtained solution was reacted at 4°C for 2 hours. Thereafter, 0.5 ml of the reaction solution was added to a Microcon filter (YM-10 manufactured by Millipore) placed on the microtube, and it was then centrifuged at 4°C at 14000 g for 60 minutes. A solution dropped into the microtube was discarded, and 0.2 ml of 1 × PBS (pH 7.4) was placed on the filter, followed by centrifugation at 4°C at 14000 g for 30 minutes. A solution dropped into the microtube was discarded, and 0.2 ml of 1 × PBS (pH 7.4) was placed on the filter, followed by centrifugation at 4°C at 14000 g for 30 minutes. Thereafter, the filter was turned upside down, and it was then set in a new microtube. It was then centrifuged at 4°C at 1000 g for 3 minutes, and a solution remaining on the filter was recovered. Further, 0.1 ml of 1 × PBS (pH 7.4) was placed in the filter, and it was then centrifuged at 4°C at 1000 g for 3 minutes. The recovered solutions were gathered to obtain a biotinylated CA solution. By operation of the Microcon filter, an unreacted biotinylating reagent was eliminated. The recovery rate of protein was approximately 70%.

### (6) Binding reaction of biotinylated CA to NeutrAvidin-immobilized substrate

The substrate prepared in (4) above, on which Neutravidin had been immobilized, was equipped in a surface plasmon resonance apparatus. 1 × PBS (pH 7.4) was poured therein, and it was then left at rest for 10 minutes. Thereafter, a baseline was confirmed (using a resonance angle after 10 minutes as a reference point). 1 × PBS (pH 7.4) solution of 0.2 mg/ml biotinylated CA (prepared in (5) above) was poured therein, and it was then left at rest for 30 minutes. After it had been left at rest, 1 × PBS (pH 7.4) was poured therein, and it was then left at rest for 1 minute. A difference between the resonance angle obtained at this time and the resonance angle of the origin was defined as the amount of biotinylated CA bound. The bound amount was found to be 7450 RV.

Similarly, the concentration of biotinylated CA was set at 0.05 mg/ml and 0.02 mg/ml, and such biotinylated CA was then treated, so as to obtain the amount of biotinylated CA immobilized of 7000 RV and 1600 RV, respectively.

### Comparative example 1 Binding reaction of compound to biotinylated CA-immobilized substrate

The substrate prepared in (6) above, on which biotinylated CA had been immobilized, was equipped in a surface plasmon resonance apparatus. Buffer solutions and compound solutions were alternatively poured therein in the following order. The binding signal of each compound was obtained from a difference with the signal of a buffer solution poured immediately before.
0. 1 × PBS (pH 7.4)
1. 100 nM Hydrochlorothiazide (SIGMA) in 1 × PBS (pH 7.4)
2. 1 × PBS (pH 7.4)
3. 100 nM Nicotinic acid (SIGMA) in 1 × PBS (pH 7.4)
4. 1 × PBS (pH 7.4)
5. 100 nM Nicotinic acid (SIGMA) in 1 × PBS (pH 7.4)
6. 1 × PBS (pH 7.4)
7. 100 nM Nicotinic acid (SIGMA) in 1 × PBS (pH 7.4)
8. 1 × PBS (pH 7.4)
9. 100 nM Nicotinic acid (SIGMA) in 1 × PBS (pH 7.4)
10. 1 × PBS (pH 7.4)
11. 100 nM Nicotinic acid (SIGMA) in 1 × PBS (pH 7.4)
12. 1 × PBS (pH 7.4)
13. 100 nM Nicotinic acid (SIGMA) in 1 × PBS (pH 7.4)
14. 1 × PBS (pH 7.4)
15. 100 nM Nicotinic acid (SIGMA) in 1 × PBS (pH 7.4)
16. 1 × PBS (pH 7.4)
17. 100 nM Nicotinic acid (SIGMA) in 1 × PBS (pH 7.4)
18. 1 × PBS (pH 7.4)
19. 100 nM Nicotinic acid (SIGMA) in 1 × PBS (pH 7.4)
20. 1 × PBS (pH 7.4)
21. 100 nM Nicotinic acid (SIGMA) in 1 × PBS (pH 7.4)
22. 1 × PBS (pH 7.4)
23. 100 nM Nicotinic acid (SIGMA) in 1 × PBS (pH 7.4)

Hydrochlorothiazide was used herein as a positive control of CA, and the binding signal was observed. Based on the assumption that CA binds to Hydrochlorothiazide via a one-to-one binding, molecular weight conversion was carried out, and the binding activity of Hydrochlorothiazide was obtained. In addition, Nicotinic acid was used as a negative control, and no binding was observed in all the substrates. The amount of Hydrochlorothiazide binding, binding activity, mean value, and CV value in each sample are shown in Table 1.

### Example 2 Approximate calibration of baseline

The absolute values of signals (12 points) in each buffer (1 × PBS) obtained in Comparative example 1 were plotted. As a result, it was revealed that the values gradually shifted to negative values (Figure 1). Such points were approximated in a third curve, using the "addition of approximate curve" function of Microsoft Excel. A difference with this approximation formula was obtained from each signal, so as to obtain calibration data. Based on the calibration data, the binding signal and binding activity of each compound were obtained in the same manner as described above. The amount of Hydrochlorothiazide binding, binding activity, mean value, and CV value of each sample are shown in Table 1.

The amount of signals subjected to the calibration of the present invention has a deviation smaller than that of the comparative example. Since a minus drift probably caused by elimination of biotinylated CA was generated, it is considered that the amount of Hydrochlorothiazide bound varies depending on the degree of drift. It is found that, according to the present invention, even if substrates with different degrees of drifts are used, data can be obtained with a small deviation among the substrates.

**Table 1**

| Comparative example (without calibration) | | | | |
|---|---|---|---|---|
| Amount of CA immobilized (RV) | Amount of 100 nM Hydrochlorothiazide bound (RV) | Binding activity (%) | Mean binding activity (%) | CV binding activity (%) |
| 7450 | 25.4 | 34.4 | 39.9 | 13.1 |
| 3400 | 13.7 | 40.6 | | |
| 1600 | 7.1 | 44.7 | | |

| Example (with calibration) | | | | |
|---|---|---|---|---|
| Amount of CA immobilized (RV) | Amount of 100 nM Hydrochlorothiazide bound (RV) | Binding activity (%) | Mean binding activity (%) | CV binding activity (%) |
| 7450 | 31.7 | 42.8 | 45.6 | 5.9 |
| 3400 | 16.3 | 48.2 | | |
| 1600 | 7.3 | 45.9 | | |

## Claims

1. A method for measuring the interaction between a physiologically active substance immobilized on the surface of a substrate and a test substance, wherein a calibration curve is produced by using baseline values obtained by repeatedly measuring a single type of solution, and the measurement value of said test substance is calibrated by said calibration curve, so as to obtain the interaction signals of said test substance.

2. The method according to claim 1, wherein the calibration curve is a curve obtained by approximating the baseline values by the least square method.

3. The method according to claim 1 or 2, wherein the calibration curve is a first exponential curve (a linear approximation), a second exponential curve, a third exponential curve or an exponential approximation.

4. The method according to claim 1, 2 or 3, wherein the number of the baseline values used in production of the calibration curve is between 4 and 1,000.

5. The method according to any one of claims 1 to 4, wherein the interaction between the physiologically active substance and the test substance is detected as a change in refractive index.

6. The method according to any one of claims 1 to 5, wherein the interaction between the physiologically active substance and the test substance is detected by surface resonance plasmon analysis.
